# EUROPEAN PATENT APPLICATION

(11) **EP 3 248 535 A1**
(43) Date of publication of application: **29.11.2017**
(21) Application number: 15878854.7
(22) Date of filing: 09.09.2015
(51) Int. Cl.: A61B 1/04, G02B 23/24

(54) **ENDOSCOPIC SYSTEM**

(30) Priority: 22.01.2015 JP 2015010556
(71) Applicant: Olympus Corporation, Shibuya-ku Tokyo 151-0072 (JP)
(72) Inventor: URAKAWA, Tsutomu, Tokyo 192-8507 (JP); KINOUCHI, Hideaki, Tokyo 192-8507 (JP); MIKAMI, Takamasa, Tokyo 192-8507 (JP)
(74) Representative: Gunzelmann, Rainer
(86) International application number: PCT/JP2015/075635
(87) International publication number: WO 2016/117165

(57) **Abstract**

Provided is an endoscope system that can accurately determine a transmission state based on a bit error rate and light receiving sensitivity, and thus is capable of coping quickly even when failure occurs in the transmission state. The endoscope system 1 includes an image sensor 10 configured to capture images of a subject, a first photoelectric sensor 11 configured to convert an imaging signal and test data output from the image sensor 10 into an optical signal and output the optical signal, an optical fiber 13 configured to transmit the optical signal output from the first photoelectric sensor 11, and an information processing device 3 including a second photoelectric sensor 20 configured to receive the optical signal transmitted through the optical fiber 13, a light amount measuring unit 22 configured to measure a receiving sensitivity of the test data converted into an electric signal by the second photoelectric sensor 20, a bit error rate measuring unit 26 configured to measure a bit error rate of the test data, and a determination unit 27 configured to determine a transmission state of the test data based on the receiving sensitivity and the bit error rate.

## Description

### Field

The present invention relates to an endoscope system for transmitting image information by an optical transmission system.

### Background

In the medical field, endoscope systems have been conventionally used when observing organs of subjects such as patients. An endoscope system includes, for example, an endoscope and an information processing device. The endoscope includes a flexible, elongated insertion portion which is provided with an image sensor at the distal end thereof and to be inserted into a body cavity of a subject. The information processing device is connected to the insertion portion through cables and connectors, performs image processing on an in-vivo image captured by the image sensor, and causes a display device to display the in-vivo image.

In recent years, image sensors having a large number of pixels that enable a clearer image observation have been developed, and the use of the image sensors having a large number of pixels for the endoscopes have been studied. In consideration of ease of insertion into a subject, there is a demand for the reduced diameter of the insertion portion. Furthermore, to transmit a large amount of signals at high speed between the image sensor and the information processing device while realizing the reduced diameter of the insertion portion, a transmission system using an optical fiber and an optical waveguide is also adopted by the endoscope system.

Among the endoscope systems using optical fibers and optical waveguides, an endoscope system disclosed performs transmission after transmitting test data to detect a transmission state in an optical fiber and an optical waveguide and checking the transmission state by a bit error rate of received test data (For example, refer to Patent Literature 1).

### Citation List

### Patent Literature

Patent Literature 1: JP 2009-061032 A

### Summary

### Technical Problem

In Patent Literature 1, however, since the transmission state is checked by the bit error rate only, it is difficult to locate where in the optical transmission path failure occurs, for example, in an optical connector portion, an optical fiber, or a photoelectric sensor portion. As a result, when the transmission state is deteriorated, it may take time to recover the transmission state.

The present invention has been made in view of the foregoing, and an object of the present invention is to provide an endoscope system that is capable of accurately determining a transmission state based on a bit error rate and light receiving sensitivity, thereby quickly correcting failure occurred in the transmission state.

### Solution to Problem

In order to solve the above described problem and achieve the object, an endoscope system according to the invention includes: an image sensor configured to capture images of a subject; a first photoelectric sensor configured to convert an imaging signal and test data output from the image sensor into an optical signal and output the optical signal; optical fibers configured to transmit the optical signal output from the first photoelectric sensor; and an information processing device including: a second photoelectric sensor configured to receive the optical signal transmitted through the optical fibers; a light amount measuring unit configured to measure a receiving sensitivity of the test data converted into an electric signal by the second photoelectric sensor; a bit error rate measuring unit configured to measure a bit error rate of the test data; and a determination unit configured to determine a transmission state of the test data based on the receiving sensitivity and the bit error rate.

The endoscope system according to the invention further includes a display device configured to display a transmission level of the test data and/or a command for recovering the transmission level.

The endoscope system according to the invention further includes: a first optical connector for holding an end portion of one of the optical fibers connected to the first photoelectric sensor; a second optical connector for holding an end portion of the other of the optical fibers connected to the second photoelectric sensor; a first connector for housing the first optical connector, the first connector being provided at an endoscope to hold an electric cable for transmitting the electric signal to the endoscope; and a second connector for housing the second optical connector, the second connector being provided at the information processing device to hold the electric cable for transmitting the electric signal to the endoscope. The first connector includes a transmission level storage unit configured to store a transmission level of the test data.

The endoscope system according to the invention further includes a current controller configured to amplify driving current of the first photoelectric sensor.

### Advantageous Effects of Invention

According to the present invention, a transmission state is determined based on a bit error rate and light receiving sensitivity in an endoscope system using an optical fiber and an optical waveguide. Therefore, it is possible to easily locate where failure has occurred, and thus correct the failure in a short period of time.

### Brief Description of Drawings

FIG. 1 is a schematic view illustrating a schematic configuration of an endoscope system according to a first embodiment of the present invention.
FIG. 2 is a block diagram of a main part of the endoscope system according to the first embodiment of the present invention.
FIG. 3 is a comparison table for determining a transmission state of the endoscope system according to the first embodiment of the present invention.
FIG. 4 is a block diagram of a main part of an endoscope system according to a modification of the first embodiment of the present invention.
FIG. 5 is a block diagram of a main part of an endoscope system according to a second embodiment of the present invention.

### Description of Embodiments

Reference will be made below to an endoscope system as modes for carrying out the present invention (hereinafter, referred to as "embodiment(s)"). The invention is not limited by the embodiments. The same reference signs are used to designate the same elements throughout the drawings.

### (First Embodiment)

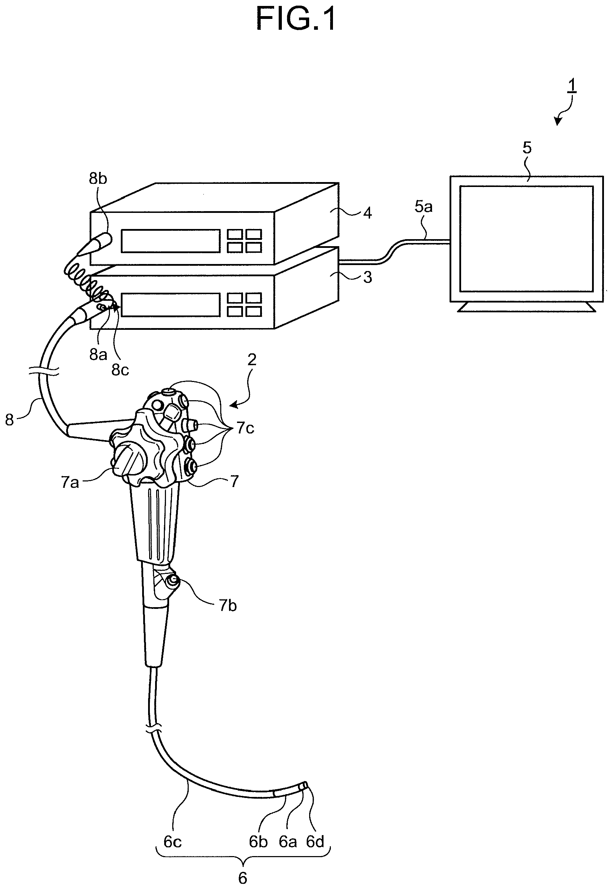

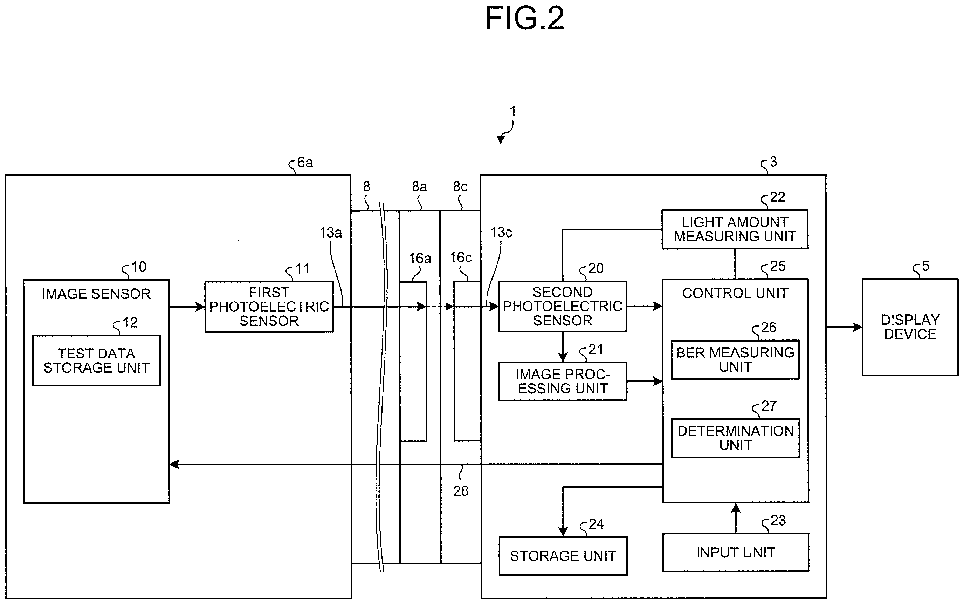

FIG. 1 is a schematic view illustrating a schematic configuration of an endoscope system according to the first embodiment of the present invention. FIG. 2 is a block diagram of a main part of the endoscope system according to the first embodiment of the present invention.

As illustrated in FIGS. 1 and 2, an endoscope system 1 according to the first embodiment includes an endoscope 2, an information processing device 3 (external processor), a light source device 4, and a display device 5. The endoscope 2 is inserted into a subject to capture an image inside a body of the subject and generate an in-vivo image signal of the subject. The information processing device 3 (external processor) performs predetermined image processing on the image signal captured by the endoscope 2, and controls each unit of the endoscope system 1. The light source device 4 generates illumination light of the endoscope 2. The display device 5 displays the image signal after the image processing by the information processing device 3.

The endoscope 2 includes an insertion portion 6, an operating unit 7, and a universal cord 8. The insertion portion 6 is configured to be inserted into the subject. The operating unit 7 is on the proximal end portion side of the insertion portion 6 and grasped by an operator. The universal cord 8 has flexibility and extends from the operating unit 7.

The insertion portion 6 is realized by using an illumination fiber (light guide cable), an electric cable 28, optical fibers 13a and 13b, and the like. The insertion portion 6 includes a distal end portion 6a, a bending portion 6b, and a flexible tube portion 6c. An image sensor 10, which will be described later, is provided in the distal end portion 6a. The bending portion 6b is bendable and includes a plurality of bending pieces. The flexible tube portion 6c is provided on the proximal end side of the bending portion 6b. The distal end portion 6a is provided with an illumination portion, an observation portion, an opening portion 6d, and an air/water supply nozzle (not illustrated). The illumination portion illuminates the inside of the subject through an illumination lens. The observation portion captures an image of the inside of the subject. The opening portion 6d communicates with a treatment tool channel.

The distal end portion 6a includes the image sensor 10 and a first photoelectric sensor 11. The image sensor 10 is provided at an image-forming position in an optical system for collecting light to receive light collected by the optical system, photoelectrically convert the light into an electric signal, and perform predetermined signal processing. The first photoelectric sensor 11 converts the electric signal including image information input from the image sensor 10 into an optical signal, and transmits the optical signal to the information processing device 3.

The image sensor 10 includes a test data storage unit 12 for storing test data. When the endoscope system 1 is powered on or when a command signal is input through the information processing device 3 which will be described later, the image sensor 10 transmits the test data stored in the test data storage unit 12 to the information processing device 3 through the first photoelectric sensor 11 to check the transmission state of the signal to be transmitted from the first photoelectric sensor 11.

The operating unit 7 includes a bending knob 7a, a treatment tool insertion portion 7b, and a plurality of switch portions 7c. The bending knob 7a causes the bending portion 6b to be bent in the vertical direction and the horizontal direction. A treatment tool, such as biopsy forceps and a laser scalpel, is configured to be inserted through the treatment tool insertion portion 7b. The plurality of switch portions 7c operates peripheral devices such as the information processing device 3, the light source device 4, an air supply device, a water supply device, and a gas supply device. The treatment tool inserted from the treatment tool insertion portion 7b is exposed from the opening portion 6d at the distal end of the insertion portion 6 through the treatment tool channel provided inside.

The universal cord 8 includes the illumination fiber, the electric cable 28, the optical fibers 13a and 13b, and the like. The universal cord 8 is branched at the proximal end thereof. One branched end portion is a first connector 8a, and the other proximal end is an illumination connector 8b. The first connector 8a is attachable to and detachable from a second connector 8c of the information processing device 3. The illumination connector 8b is attachable to and detachable from the light source device 4. Furthermore, as illustrated in FIG. 2, the first connector 8a houses a first optical connector 16a for holding the optical fiber 13a. The second connector 8c houses a second optical connector 16c for holding an optical fiber 13c. The image signal captured by the image sensor 10 provided in the distal end portion 6a is transmitted to the information processing device 3 through the first photoelectric sensor 11, the optical fiber 13a, the optical fiber 13c, and the optical signal (indicated by a dotted line in FIG. 2) connecting the optical fiber 13a and the optical fiber 13c.

The information processing device 3 includes a second photoelectric sensor 20, an image processing unit 21, a light amount measuring unit 22, an input unit 23, a storage unit 24, and a control unit 25. The second photoelectric sensor 20 converts the optical signal including the image information transmitted from the first photoelectric sensor 11 into an electric signal. The image processing unit 21 generates an in-vivo image to be displayed on the display device 5 based on the image information input from the second photoelectric sensor 20. The light amount measuring unit 22 measures the receiving sensitivity of the optical signal received by the second photoelectric sensor 20. The input unit 23 inputs various kinds of signals such as operation command signals for instructing the operations of the endoscope system 1. The storage unit 24 stores various kinds of programs for operating the endoscope system 1. The control unit 25 controls driving of each unit as well as inputs and outputs of information in each unit. In addition, the control unit 25 includes a BER measuring unit 26 and a determination unit 27. The BER measuring unit 26 measures a bit error rate (hereinafter, also referred to as "BER") of the test data received by the second photoelectric sensor 20. The determination unit 27 determines the transmission state based on the receiving sensitivity measured by the light amount measuring unit 22 and the BER measured by the BER measuring unit 26.

The light source device 4 includes a light source that emits light, a condenser lens, and the like. Under the control of the information processing device 3, the light source device 4 emits and supplies light from the light source to the endoscope 2 connected through the illumination connector 8b, the illumination fiber of the universal cord 8, and the first connector 8a, as light for illuminating the inside of the subject as an object.

The display device 5 includes a display using liquid crystals or organic electro luminescence (EL), and the like. Through a video cable 5a, the display device 5 displays various kinds of information including the image after the predetermined image processing by the information processing device 3. Accordingly, the operator can observe a desired position inside the subject and determine characteristics by operating the endoscope 2 while looking at the image (in-vivo image) displayed on the display device 5.

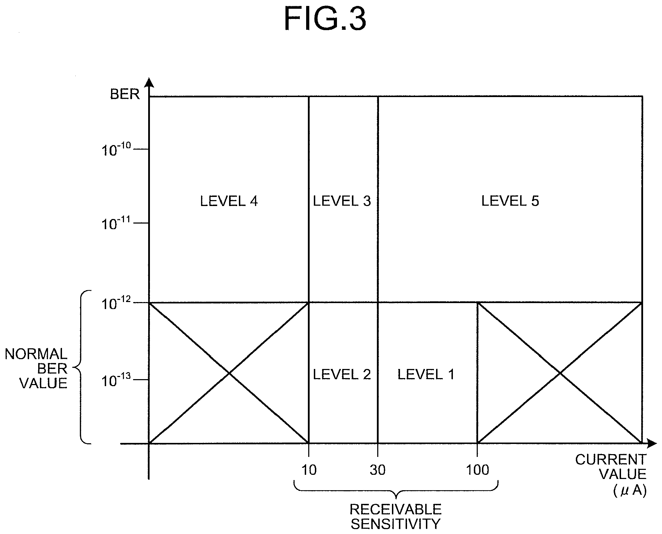

Next, a check on the transmission state in the endoscope system 1 according to the first embodiment will be described with reference to FIG. 3. FIG. 3 is a comparison table for determining the transmission state of the endoscope system according to the first embodiment of the present invention.

When the endoscope system 1 is powered on, the test data stored in the test data storage unit 12 of the image sensor 10 is output to the first photoelectric sensor 11. The first photoelectric sensor 11 converts the test data received as an electric signal into an optical signal, and transmits the optical signal to the second photoelectric sensor 20 of the information processing device 3 through the optical fiber 13a held by the first optical connector 16a, the optical signal (indicated by the dotted line in FIG. 2) connecting the optical fiber 13a and the optical fiber 13c, and the optical fiber 13c held by the second optical connector 16c. The second photoelectric sensor 20 converts the received optical signal into an electric signal, and then outputs the electric signal to each of the light amount measuring unit 22 and the BER measuring unit 26.

The light amount measuring unit 22 measures the receiving sensitivity of the test data converted into the electric signal as a current value. The BER measuring unit calculates the bit error rate (BER) of the received test data. The determination unit 27 determines, from the comparison table illustrated in FIG. 3, the transmission level of the received signal based on the receiving sensitivity measured by the light amount measuring unit 22 and the bit error rate calculated by the BER measuring unit.

In the comparison table illustrated in FIG. 3, the vertical axis represents the BER and the horizontal axis represents the current value. In FIG. 3, a normal BER value is indicated as 10⁻¹² or less and a receivable current value is indicated as 10 to 100 pA. However, these numerical values change according to the performance of the sensors used as the first photoelectric sensor 11 and second photoelectric sensor 20, and thus these numerical values are merely examples.

As illustrated in FIG. 3, the transmission level is classified into level 1 to level 5 according to the bit error rate and the receiving sensitivity. In Level 1, the BER value is 10⁻¹² or less and the receiving sensitivity is 30 µA or more and 100 pA or less. When the received test data is within this range, it is determined that there is no problem for practical use. When the transmission level is determined to be level 1, the display device 5 displays nothing or displays that the transmission level is level 1 and there is no problem for the endoscope observation.

When the BER value is 10⁻¹² or less and the receiving sensitivity is 10 µA or more and less than 30 µA, the transmission level is determined to be level 2, and the display device 5 displays that the transmission level is 2 and there is no problem for the endoscope observation. When the transmission level is level 2, it is possible to continue the endoscope observation, but there are some cases where the receiving sensitivity decreases, and failure occurs depending on the subsequent use. Accordingly, the display device 5 displays a method for recovering the transmission level. A possible reason that the transmission level decreases to level 2 is dirt of the first optical connector 16a or the second optical connector 16c, or a poor contact of the first optical connector 16a or the second optical connector 16c. Therefore, the display device 5 displays a command such as "Remove the first connector 8a (the first optical connector 16a) from the second connector 8c of the information processing device 3 and wipe the end surfaces of the first connector 8a (the first optical connector 16a) and the second connector 8c (the second optical connector 16c)", "Connect the first connector 8a (the first optical connector 16a) to the second connector 8c properly", and the like.

When the BER value is larger than 10⁻¹² and the receiving sensitivity is 10 µA or more and less than 30 µA, the transmission level is determined to be level 3, and the display device 5 displays that the transmission level is 3 and there is a problem for the endoscope observation. When the transmission level is level 3, there is a problem in continuing the endoscope observation. Therefore, in a case where the transmission level is not recovered by the method displayed, the endoscope observation is stopped. A possible reason that the transmission level decreases to the level 3 is dirt of the first optical connector 16a or the second optical connector 16c, a poor connection therebetween, a breakage of the optical fibers 13a and 13b, or a failure of the first photoelectric sensor 11. As a method that the operator can carry out, the display device 5 displays a command such as "Remove the first connector 8a (the first optical connector 16a) from the second connector 8c of the information processing device 3 and wipe the end surfaces of the first connector 8a (the first optical connector 16a) and the second connector 8c (the second optical connector 16c)", "Connect the first electrical connector 8a (the first optical connector 16a) to the second connector 8c properly", and the like. When the operator cleans the end surfaces of the first connector 8a (the first optical connector 16a) and the second connector 8c (the second optical connector 16c), and the first connector 8a (the first optical connector 16a) is reconnected to the second connector 8c, the test data is transmitted from the image sensor 10 again, and the transmission level is determined.

When the BER value is larger than 10⁻¹² and the receiving sensitivity is less than 10 µA, the transmission level is determined to be level 4, and the display device 5 displays that the transmission level is 4 and there is a problem for the endoscope observation. When the transmission level is level 4, the most likely cause is the decrease in the amount of light due to the breakage of the optical fibers 13a and 13b and it is difficult to recover the transmission level by the operator. Therefore, the necessity of the inspection of the endoscope 2 is displayed on the display device 5.

When the BER value is larger than 10⁻¹² and the receiving sensitivity is larger than 30 µA, the transmission level is determined to be level 5, and the display device 5 displays that the transmission level is 5 and there is a problem for the endoscope observation. When the transmission level is level 5, the most likely cause is the failure of the first photoelectric sensor 11, and as in the case of level 4, it is difficult to recover the transmission level by the operator. Therefore, the necessity of the inspection of the endoscope 2 is displayed on the display device 5.

When the transmission level is determined to be level 3 to level 5, the endoscope 2 is sent out to a service center or the like for inspection. With the transmission level, the handling at the service center becomes easy.

The endoscope system 1 according to the first embodiment can accurately determine the transmission state based on the bit error rate and the light receiving sensitivity. Therefore, the transmission level of the endoscope can be recovered by the operator or at the service center easily.

In the first embodiment, the transmission level is classified into levels according to the bit error rate and receiving sensitivity, and the display device 5 displays a command for recovering the transmission level. Alternatively, by providing a storage unit for storing the transmission level in the endoscope, the display device 5 may only display the command for recovering the transmission level without displaying the transmission level on the display device.

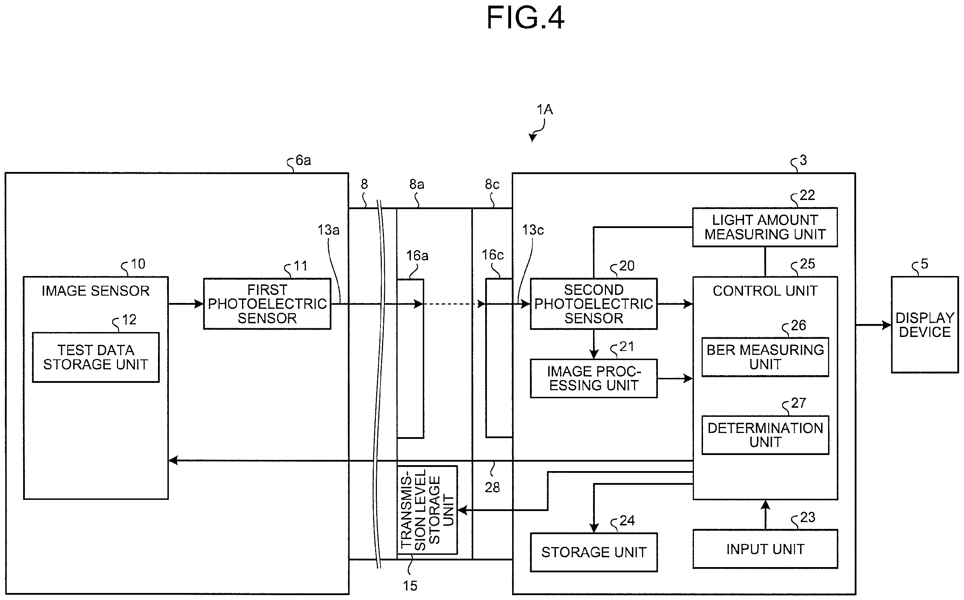

FIG. 4 is a block diagram of a main part of an endoscope system according to a modification of the first embodiment of the present invention. In an endoscope system 1A according to the modification, a first connector 8a includes a transmission level storage unit 15 that stores the transmission level of test data.

The transmission level storage unit 15 stores a transmission level or actual values of the bit error rate and the receiving sensitivity for determining the transmission state. When the transmission level decreases, a display device 5 displays a command for recovering the transmission level. In a case where the transmission level is not recovered even with the command, an endoscope 2 is sent out to a service center for inspection. Based on the transmission level or the actual values of the bit error rate and the receiving sensitivity stored in the transmission level storage unit 15, the service center can determine a location of the failure and make a repair quickly. The transmission level storage unit 15 storing the transmission level or the actual values of the bit error rate and the receiving sensitivity for determining the transmission state may be provided in a second connector 8c, or the transmission level or the actual values of the bit error rate and the receiving sensitivity for determining the transmission state may be stored in a storage unit 24 inside an information processing device 3, without providing the transmission level storage unit 15.

Although the image information captured by the image sensor 10 is transmitted to the information processing device 3 through a single transmission line (single first photoelectric sensor 11, optical fiber 13a, first optical connector 16a, optical signal, second optical connector 16c, and optical fiber 13c) in the first embodiment, the image information may be transmitted through two transmission lines (two first photoelectric sensors and optical fibers) to transmit more image information while reducing the amount of heat generation. If the image information is transmitted through the two transmission lines, the transmission level is determined and displayed on the display device 5 for each of the lines. When the transmission level of one of the two transmission lines decreases, the other transmission line whose transmission level does not decrease can be used to transmit all the image information, as long as it is in a short period of time.

### (Second Embodiment)

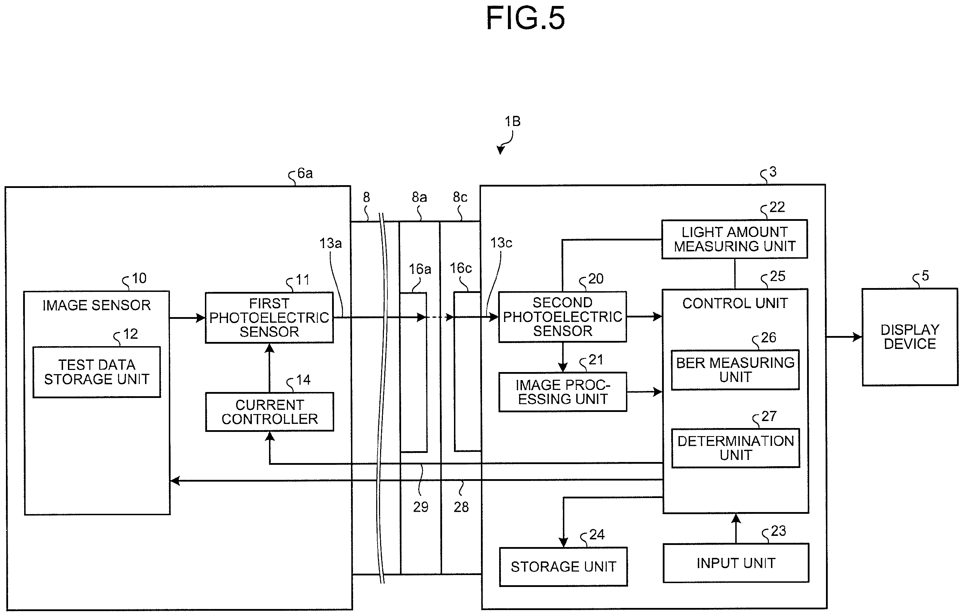

FIG. 5 is a block diagram of a main part of an endoscope system according to the second embodiment of the present invention. An endoscope system 1B according to the second embodiment includes a current controller 14 for amplifying the driving current of a first photoelectric sensor 11. When the receiving sensitivity is low, the current controller 14 amplifies the driving current of the first photoelectric sensor 11, whereby the output of an optical signal to be transmitted by the first photoelectric sensor 11 increases.

As in the first embodiment, when the endoscope system 1B is powered on, test data stored in a test data storage unit 12 of an image sensor 10 is output to the first photoelectric sensor 11. After the test data is converted into an optical signal by the first photoelectric sensor 11, the optical signal is transmitted to a second photoelectric sensor 20 of an information processing device 3 through an optical fiber 13a held by a first optical connector 16a, the optical signal (indicated by a dotted line in FIG. 5) connecting the optical fiber 13a and an optical fiber 13c, and the optical fiber 13c held by a second optical connector 16c. After the second photoelectric sensor 20 converts the received optical signal into an electric signal, the second photoelectric sensor 20 outputs the electric signal to each of a light amount measuring unit 22 and a BER measuring unit 26, and the receiving sensitivity and the bit error rate are measured.

In the second embodiment, when the receiving sensitivity measured by the light amount measuring unit 22 is lower than a predetermined value, a control unit 25 outputs a control signal for amplifying the driving current to the current controller 14 through an electric cable 29. When the receiving sensitivity is larger than the predetermined value, for example in case of level 5, a display device 5 displays that there is a problem for the endoscope observation.

When the control signal is input to the current controller 14, the current controller 14 amplifies the driving current of the first photoelectric sensor 11, whereby the output of the optical signal to be transmitted by the first photoelectric sensor 11 increases.

After the output of the optical signal to be transmitted by the first photoelectric sensor 11 is increased by the current controller 14, the test data is transmitted again from the image sensor 10, and the transmission level is determined. When the output of the optical signal increases, the receiving sensitivity increases, and at the same time, the BER decreases.

In a case where the transmission level is not recovered even by the increased output level of the optical signal, the display device 5 displays, as in the first embodiment, a corresponding transmission level and a command for recovering the transmission level.

In the second embodiment, when the receiving sensitivity decreases, the output of the optical signal transmitted by the first photoelectric sensor can be increased by the current controller. Therefore, even when the decrease in the transmission level is observed, an image with less noise can be obtained.

### Reference Signs List

1 ENDOSCOPE SYSTEM
2 ENDOSCOPE
3 INFORMATION PROCESSING DEVICE
4 LIGHT SOURCE DEVICE
5 DISPLAY DEVICE
6 INSERTION PORTION
6a DISTAL END PORTION
6b BENDING PORTION
6c FLEXIBLE TUBE PORTION
6d OPENING PORTION
7 OPERATING UNIT
7a BENDING KNOB
7b TREATMENT TOOL INSERTION PORTION
7c SWITCH PORTIONS
8 UNIVERSAL CORD
8a FIRST CONNECTOR
8b ILLUMINATION CONNECTOR
8c SECOND CONNECTOR
10 IMAGE SENSOR
11 FIRST PHOTOELECTRIC SENSOR
12 TEST DATA STORAGE UNIT
13a, 13c OPTICAL FIBER
14 CURRENT CONTROLLER
15 TRANSMISSION LEVEL STORAGE UNIT
16a FIRST OPTICAL CONNECTOR
16c SECOND OPTICAL CONNECTOR
20 SECOND PHOTOELECTRIC SENSOR
21 IMAGE PROCESSING UNIT
22 LIGHT AMOUNT MEASURING UNIT
23 INPUT UNIT
24 STORAGE UNIT
25 CONTROL UNIT
26 BER MEASURING UNIT
27 DETERMINATION UNIT
28, 29 ELECTRIC CABLE

## Claims

1. An endoscope system comprising:
an image sensor configured to capture images of a subject;
a first photoelectric sensor configured to convert an imaging signal and test data output from the image sensor into an optical signal and output the optical signal;
optical fibers configured to transmit the optical signal output from the first photoelectric sensor; and
an information processing device comprising:
a second photoelectric sensor configured to receive the optical signal transmitted through the optical fibers;
a light amount measuring unit configured to measure a receiving sensitivity of the test data converted into an electric signal by the second photoelectric sensor;
a bit error rate measuring unit configured to measure a bit error rate of the test data; and
a determination unit configured to determine a transmission state of the test data based on the receiving sensitivity and the bit error rate.

2. The endoscope system according to claim 1, further comprising:
a display device configured to display a transmission level of the test data and/or a command for recovering the transmission level.

3. The endoscope system according to claim 1 or 2, further comprising:
a first optical connector for holding an end portion of one of the optical fibers connected to the first photoelectric sensor;
a second optical connector for holding an end portion of the other of the optical fibers connected to the second photoelectric sensor;
a first connector for housing the first optical connector, the first connector being provided at an endoscope to hold an electric cable for transmitting the electric signal to the endoscope; and
a second connector for housing the second optical connector, the second connector being provided at the information processing device to hold the electric cable for transmitting the electric signal to the endoscope, and
wherein the first connector comprises a transmission level storage unit configured to store a transmission level of the test data.

4. The endoscope system according to any one of claims 1 to 3, further comprising:
a current controller configured to amplify driving current of the first photoelectric sensor.
